# EUROPÄISCHE PATENTANMELDUNG

(11) **EP 3 270 138 A1**
(43) Veröffentlichungstag der Anmeldung: **17.01.2018**
(21) Anmeldenummer: 17179723.6
(22) Anmeldetag: 05.07.2017
(51) Int. Cl.: G01N 7/00, A61L 2/26, G01N 25/60

(54) **ANALYSEVORRICHTUNG UND VERFAHREN ZUM ANALYSIEREN VON DAMPF ZUM STERILISIEREN FÜR EINE STERILISIERVORRICHTUNG UND STERILISIERVORRICHTUNG**

(30) Priorität: 11.07.2016 DE 102016112631
(71) Anmelder: Miele & Cie. KG, 33332 Gütersloh (DE)
(72) Erfinder: GREGOR, Olaf, 83373 Taching (DE); ÖHLINGER, Andreas, 5110 Oberndorf (AT)

(57) **Zusammenfassung**

Der hier vorgestellte Ansatz betrifft eine Analysevorrichtung (100) zum Analysieren eines Dampfs zum Sterilisieren für eine Sterilisiervorrichtung. Die Analysevorrichtung (100) weist zumindest eine Kühleinrichtung (105) zum Kühlen einer Dampfmenge des Dampfs zum Bewirken einer Kondensation zum Erhalten eines Kondensat-Gas-Gemischs (135) auf. Weiterhin weist die Analysevorrichtung (100) eine Aufnahmeeinrichtung (110) zum Aufnehmen des Kondensat-Gas-Gemischs (135) auf, wobei die Aufnahmeeinrichtung (110) mit der Kühleinrichtung (105) gekoppelt ist. Letztlich weist die Analysevorrichtung (100) eine Messeinrichtung (115) zum Messen einer Gemischmasse des Kondensat-Gas-Gemischs (135) auf, wobei die Messeinrichtung (115) mit der Aufnahmeeinrichtung (110) gekoppelt ist.

## Beschreibung

Der hier vorgestellte Ansatz betrifft eine Analysevorrichtung und ein Verfahren zum Analysieren von Dampf zum Sterilisieren für eine Sterilisiervorrichtung und eine Sterilisiervorrichtung.

Laut DIN EN 285 darf der Anteil von nichtkondensierbaren Gasen in einem Sterilisationsdampf 3,5% bezogen auf das Kondensatvolumen nicht überschreiten. Darum ist es wichtig, dass vor dem Sterilisieren kontinuierlich ein Nachweis der Dampfqualität erbracht wird.

Die DE 43 19 402 A1 beschreibt eine Vorrichtung, die als Messprinzip eine Massereduktion von Wasser durch gelöste Gasanteile verwendet. Zur Reduzierung des Einflusses des Stokes'schen Auftriebs ist hierbei der Durchmesser eines Steigrohres sehr klein gewählt. Der zu messende Druck der Wassersäule ist dadurch sehr gering, was zu hohen Ungenauigkeiten führen kann; eine erforderliche Messzelle benötigt eine enorm hohe Genauigkeit und ist dadurch sehr teuer; der Umgebungsdruck muss über eine Kompensationsberechnung berücksichtigt werden.

Dem hier vorgestellten Ansatz liegt die Aufgabe zugrunde, eine verbesserte Analysevorrichtung zum Analysieren von Dampf für eine Sterilisiervorrichtung, eine verbesserte Sterilisiervorrichtung, insbesondere für medizinische Instrumente, sowie ein verbessertes Verfahren zum Analysieren von Dampf für eine Sterilisiervorrichtung zu schaffen.

Erfindungsgemäß wird diese Aufgabe durch eine Analysevorrichtung zum Analysieren von Dampf zum Sterilisieren, ferner eine Sterilisiervorrichtung und schließlich ein Verfahren zum Analysieren von Dampf zum Sterilisieren mit den Merkmalen bzw. Schritten der Hauptansprüche gelöst. Vorteilhafte Ausgestaltungen und Weiterbildungen des Ansatzes ergeben sich aus den nachfolgenden Unteransprüchen.

Es wird eine Analysevorrichtung zum Analysieren eines Dampfs zum Sterilisieren für eine Sterilisiervorrichtung vorgestellt. Die Analysevorrichtung weist zumindest eine Kühleinrichtung zum Kühlen einer Dampfmenge des Dampfs zum Bewirken einer Kondensation zum Erhalten eines Kondensat-Gas-Gemischs auf. Weiterhin weist die Analysevorrichtung eine Aufnahmeeinrichtung zum Aufnehmen des Kondensat-Gas-Gemischs auf, wobei die Aufnahmeeinrichtung mit der Kühleinrichtung gekoppelt ist. Die Aufnahmeeinrichtung kann dabei dazu ausgeformt sein, um ein festgelegtes Volumen des Kondensat-Gas-Gemischs aufzunehmen. Letztlich weist die Analysevorrichtung eine Messeinrichtung zum Messen einer Gemischmasse des Kondensat-Gas-Gemischs auf, wobei die Messeinrichtung mit der Aufnahmeeinrichtung gekoppelt ist.

Eine hier vorgestellte Analysevorrichtung hat den Vorteil, dass auf sehr einfache Weise, nämlich durch das Messen der Masse des Kondensat-Gas-Gemischs eine erforderliche Rechengröße zum Berechnen eines Anteils von nichtkondensierbarem Gas in dem Kondensat-Gas-Gemisch bereitgestellt wird.

Wenn die Analysevorrichtung gemäß einem Ausführungsbeispiel zusätzlich eine Bestimmeinrichtung zum Bestimmen eines Volumenanteils des nichtkondensierbaren Gases des Kondensat-Gas-Gemischs aufweist, kann unter Verwendung der Gemischmasse und einer beispielsweise in der Bestimmungseinrichtung gespeicherten Sollmasse der Volumenanteil des nichtkondensierbaren Gases des Kondensat-Gas-Gemischs bestimmt werden. Die Sollmasse beschreibt hierbei innerhalb eines Toleranzbereichs eine Masse des Kondensat-Gas-Gemischs mit einem definierten Volumenanteil an nichtkondensierbarem Gas für das festgelegte Volumen. Der definierte Volumenanteil kann hierbei einen Volumenanteil von 0 bis 3,5 % betragen, der laut der DIN EN 285 zulässig ist. Die Sollmasse kann in diesem Fall also die Masse eines Kondensat-Gas-Gemischs beschreiben, das einen Volumenanteil von höchstens 3,5 % an nichtkondensierbarem Gas für das festgelegte Volumen aufweist. Wenn die Bestimmungseinrichtung mit der Messeinrichtung gekoppelt ist, kann die gemessene Gemischmasse unter Verwendung der Sollmasse sehr schnell Aufschluss darüber geben, ob das Kondensat-Gas-Gemisch beispielsweise einen zu hohen Volumenanteil an nichtkondensierbarem Gas aufweist. Vorteilhafterweise liegt mit der Gemischmasse ein direkt auswertbares Signal vor und Umgebungswerte wie ein Umgebungsdruck können vernachlässigt werden.

Zum Entnehmen der Dampfmenge des Dampfs aus einer Dampfleitung in die Kühleinrichtung kann die Analysevorrichtung eine Entnahmeeinrichtung aufweisen, die mit der Dampfleitung zum Führen des Dampfes gekoppelt ist. Wenn die Dampfleitung zwischen einer Dampferzeugungseinrichtung zum Erzeugen des Dampfs und einem Sterilisator zum Sterilisieren des Dampfs angeordnet ist, kann der Dampf vorteilhafterweise vor der Verwendung als Sterilisationsdampf analysiert werden.

Zum Kühlen der Dampfmenge kann die Kühleinrichtung zumindest einen Ventilator aufweisen oder mit einem Ventilator gekoppelt sein.

Gemäß einer Ausführungsform kann die Messeinrichtung mit einer Steuerung der Sterilisiervorrichtung gekoppelt oder koppelbar sein. Die Steuerung kann beispielsweise dazu ausgebildet sein, um, wie zuvor anhand der Bestimmungseinrichtung beschrieben, den Volumenanteil des nichtkondensierbaren Gases unter Verwendung der Gemischmasse und der Soll masse zu bestimmen. Zusätzlich oder alternativ kann die Steuerung auch die Verwendung des Dampfes als Sterilisationsdampf abhängig von der gemessenen Gemischmasse bzw. dem bestimmten Volumenanteil machen.

Die Aufnahmeeinrichtung kann zumindest ein Messrohr aufweisen, das zumindest teilweise spiralförmig und/oder aus Glas ausgeformt ist. Ein solches Messrohr kann dazu ausgeformt sein, um ein festgelegtes Volumen aufzunehmen.

Um die Gemischmasse besonders einfach und genau durch Wiegen zu messen, kann die Messeinrichtung zumindest eine Wägezelle aufweisen.

Die Aufnahmeeinrichtung kann alternativ auch ein einseitig eingespanntes und durchbiegbares Messrohr aufweisen, das wenn das Kondensat-Gas-Gemisch aufgenommen ist, anhand einer Durchbiegung des Messrohrs die Gemischmasse unter Verwendung eines Dehnmessstreifens der Messeinrichtung anzeigen kann.

Es ist weiterhin von Vorteil, wenn die Analysevorrichtung einen Temperatursensor aufweist, der dazu ausgebildet ist, um eine Temperatur des Kondensat-Gas-Gemischs zu sensieren. So kann ein Temperatureinfluss auf die Dichte des Kondensat-Gas-Gemischs kompensierbar sein. Der Temperatursensor kann vorteilhafterweise zwischen der Kühleinrichtung und der Aufnahmeeinrichtung angeordnet sein, um die Temperatur des Kondensat-Gas-Gemischs vor dem Messen zu sensieren.

Eine Sterilisiervorrichtung zum Sterilisieren, insbesondere von medizinischen Instrumenten, mittels Dampf weist eine Dampferzeugungseinrichtung zum Erzeugen des Dampfs, einen Sterilisator zum Verwenden des Dampfs zum Sterilisieren, eine Dampfleitung zum Verbinden der Dampferzeugungseinrichtung mit dem Sterilisator und eine der beschriebenen Analysevorrichtungen auf, die mit der Dampfleitung gekoppelt ist. Somit kann eine bekannte Sterilisiervorrichtung vorteilhaft mit der hier beschriebenen Analysevorrichtung ergänzt werden.

Ein Verfahren zum Analysieren eines Dampfs zum Sterilisieren für eine Sterilisiervorrichtung umfasst zumindest die folgenden Schritte:
Kühlen einer Dampfmenge des Dampfs in einer Kühleinrichtung zum Bewirken einer Kondensation zum Erhalten eines Kondensat-Gas-Gemischs;
Aufnehmen des Kondensat-Gas-Gemischs in eine Aufnahmeeinrichtung; und
Messen einer Gemischmasse des Kondensat-Gas-Gemischs unter Verwendung einer mit der Aufnahmeeinrichtung gekoppelten Messeinrichtung.

Auch durch eine solche Ausführungsform können die Vorteile des hier vorgestellten Ansatzes realisiert werden.

Ausführungsbeispiele des Ansatzes sind in den Zeichnungen rein schematisch dargestellt und werden nachfolgend näher beschrieben. Es zeigt
- Figur 1 bis 3: eine schematische Darstellung einer Analysevorrichtung zum Analysieren eines Dampfs zum Sterilisieren für eine Sterilisiervorrichtung gemäß Ausführungsbeispielen;
- Figur 4: eine schematische Darstellung einer Sterilisiervorrichtung gemäß einem Ausführungsbeispiel;
- Figur 5 bis 6: eine schematische Darstellung einer Analysevorrichtung gemäß einem Ausführungsbeispiel; und
- Figur 7: ein Ablaufdiagramm eines Verfahrens zum Analysieren eines Dampfs zum Sterilisieren gemäß einem Ausführungsbeispiel.

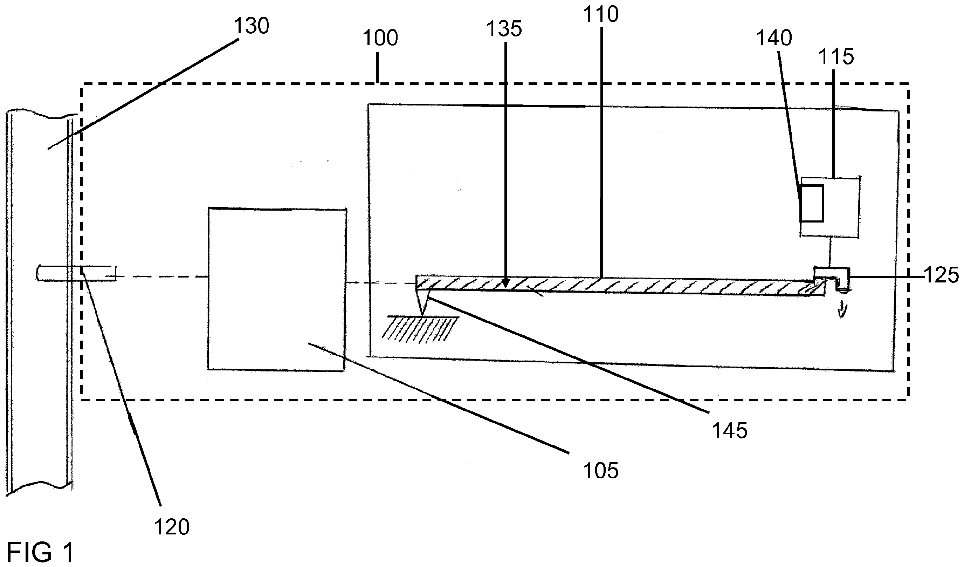

Figur 1 zeigt eine schematische Darstellung einer Analysevorrichtung 100 zum Analysieren eines Dampfs zum Sterilisieren für eine Sterilisiervorrichtung gemäß einem Ausführungsbeispiel.

Die Analysevorrichtung 100 ist dazu ausgebildet, um eine Teilmenge eines Dampfes zu analysieren, bevor oder während der verbleibende Dampf zum Sterilisieren verwendet wird. Hierzu weist die Analysevorrichtung 100 eine Kühleinrichtung 105, eine Aufnahmeeinrichtung 110 und eine Messeinrichtung 115 auf.

Optional weist die Analysevorrichtung 100 gemäß diesem Ausführungsbeispiel eine Entnahmeeinrichtung 120 und eine Auslaufeinrichtung 125 auf. Gemäß diesem Ausführungsbeispiel ist die Analysevorrichtung 100 mittels der Entnahmeeinrichtung 120 mit einer Dampfleitung 130 gekoppelt, durch die der zum Sterilisieren zu verwendende Dampf geführt wird.

Die Kühleinrichtung 105 der Analysevorrichtung 100 ist dazu ausgebildet, um eine Dampfmenge des Dampfs aufzunehmen und zu kühlen und eine Kondensation der Dampfmenge zu bewirken, um ein Kondensat-Gas-Gemisch 135 zu erhalten. Die Aufnahmeeinrichtung 110 ist dazu ausgebildet, um das entstandene Kondensat-Gas-Gemisch 135 aufzunehmen. Hierzu ist die Aufnahmeeinrichtung 110 mit der Kühleinrichtung 105 gekoppelt. Die Messeinrichtung 115 ist dazu ausgebildet, um eine Gemischmasse des Kondensat-Gas-Gemischs 135 zu messen, wobei die Messeinrichtung 115 mit der Aufnahmeeinrichtung 110 gekoppelt ist.

Gemäß diesem Ausführungsbeispiel ist die Entnahmeeinrichtung 120 dazu ausgebildet, um die Dampfmenge aus der Dampfleitung 130 zu entnehmen und in die Kühleinrichtung 105 zu führen. Die Auslaufeinrichtung 125 ist dazu ausgebildet, um das analysierte Kondensat-Gas-Gemischs 135 wieder aus der Analysevorrichtung 100 heraus zu leiten.

Die Aufnahmeeinrichtung 110 ist gemäß diesem Ausführungsbeispiel als ein gerades Messrohr ausgeformt, das ein festgelegtes Volumen an Kondensat-Gas-Gemisch 135 aufnimmt. Gemäß diesem Ausführungsbeispiel ist die Messeinrichtung 115 als eine Wägezelle 115 ausgeformt, die dazu ausgebildet ist, um die Gemischmasse des Kondensat-Gas-Gemischs 135 durch Wiegen zu messen.

Gemäß diesem Ausführungsbeispiel weist die Messeinrichtung 115 eine Bestimmeinrichtung 140 auf. Die Bestimmeinrichtung 140 ist dazu ausgebildet, um einen Volumenanteil eines nichtkondensierbaren Gases des Kondensat-Gas-Gemischs 135 unter Verwendung der Gemischmasse und einer Soll masse zu bestimmen. Die Sollmasse kann in der Bestimmeinrichtung 140 gespeichert sein und innerhalb eines Toleranzbereichs eine Masse des Kondensat-Gas-Gemischs 135 mit einem definierten Volumenanteil von beispielsweise höchstens 3,5 % an nichtkondensierbarem Gas repräsentieren. Die Bestimmeinrichtung 140 ist gemäß diesem Ausführungsbeispiel mit einer Steuerung der Sterilisiervorrichtung gekoppelt.

Gemäß einem alternativen Ausführungsbeispiel weist die Messeinrichtung 115 keine Bestimmungseinrichtung 140 auf. In diesem Fall kann eine entsprechende Bestimmungseinrichtung 140 in der Steuerung der Sterilisiervorrichtung integriert sein oder die Steuerung kann ausgebildet sein, um eine der Bestimmungseinrichtung 140 entsprechende Funktionalität umzusetzen. In diesem Fall kann die Messeinrichtung 115 signalübertragungsfähig mit der Steuerung gekoppelt sein.

Im Folgenden werden Ausführungsbeispiele anhand der Fig. 1 nochmals näher beschrieben.

Aus der Dampfleitung 130 wird über die Entnahmeeinrichtung 120 eine gewisse Menge des zu prüfenden Dampfs laufend beziehungsweise zeitweise entnommen und über die Kühleinrichtung 105 geführt. Das entstehende Kondensat-Gas-Gemisch 135 wird einer Messvorrichtung in Form der Aufnahmeeinrichtung 110 zugeführt, die gemäß diesem Ausführungsbeispiel wie folgt ausgeführt ist: Ein weitgehend waagerechtes, einseitig gelagertes Messrohr, das auch als Messleitung bezeichnet werden kann, wird mit dem Kondensat-Gas-Gemisch 135 kontinuierlich durchströmt. An einem der Kühleinrichtung 105 zugewandten Ende des Messrohrs weist das Messrohr gemäß diesem Ausführungsbeispiel ein Lager 145 auf, das dazu ausgeformt ist, um das Messrohr kippbar zu lagern. Das Messrohr erfährt durch die Masse des durchströmenden Kondensat-Gas-Gemischs 135 eine Masseänderung, welche gemäß diesem Ausführungsbeispiel mittels der Wägezelle, ausgewertet wird. Die Auslaufeinrichtung 125 des Messrohres ist so gestaltet, dass das Volumen des zu messenden Kondensat-Gas-Gemischs 135 immer gleich ist. Über die Masseänderung der Gemischmasse gegenüber der Soll masse kann ein Rückschluss auf den Gasanteil des sich jeweils im Messrohr befindlichen Kondensat-Gas-Gemischs 135 in Volumen-% gezogen werden. Die Justage der Messeinrichtung 115 kann sehr einfach z. B. mittels geeichter Prüfgewichte im Vorhinein vorgenommen worden sein.

Die vorgestellte Analysevorrichtung 100 ist vorteilhafterweise durch große Querschnitte unempfindlich gegen Verschmutzung und verbindet ein einfaches Messprinzip mit preiswerter Messtechnik. Die Analysevorrichtung 100 ist zudem leicht zu justieren und ermöglicht eine einfache Auswertung der Messwerte.

Gemäß dem gezeigten Ausführungsbeispiel ist die Aufnahmeeinrichtung 110 an einem der Kühleinrichtung 105 zugewandten ersten Ende über das Lager 145 drehbar gelagert. An dem dem ersten Ende gegenüberliegenden zweiten Ende wird die Aufnahmeeinrichtung 110 von der Messeinrichtung 115 gehalten.

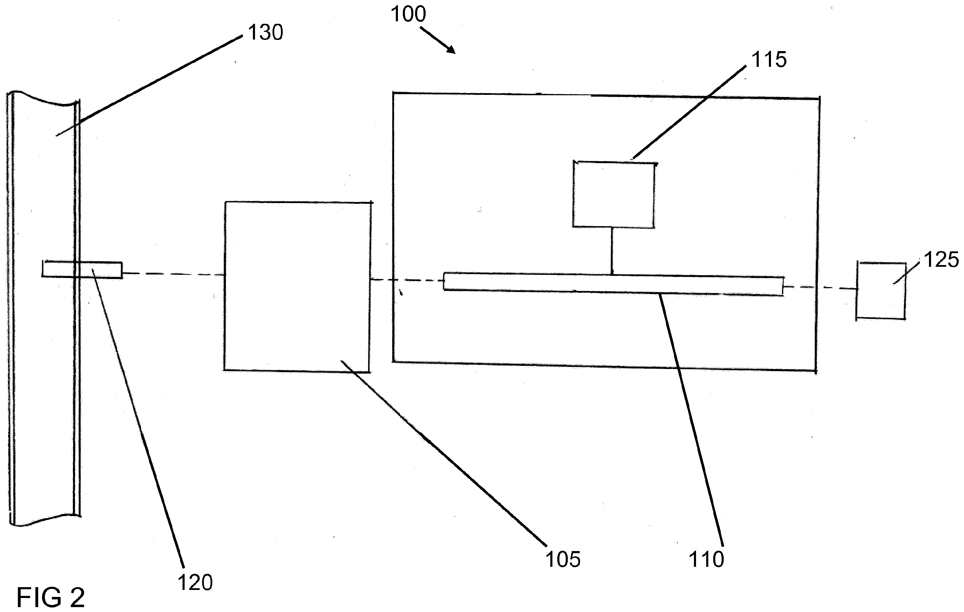

Figur 2 zeigt eine schematische Darstellung einer Analysevorrichtung 100 gemäß einem Ausführungsbeispiel. Dabei kann es sich um die anhand von Fig. 1 beschriebene Analysevorrichtung 100 handeln, mit dem Unterschied, dass die Messeinrichtung 115 mittig mit der Aufnahmeeinrichtung 110 gekoppelt ist. Auf diese Weise ist keine Lagerung der Aufnahmeeinrichtung 110 erforderlich.

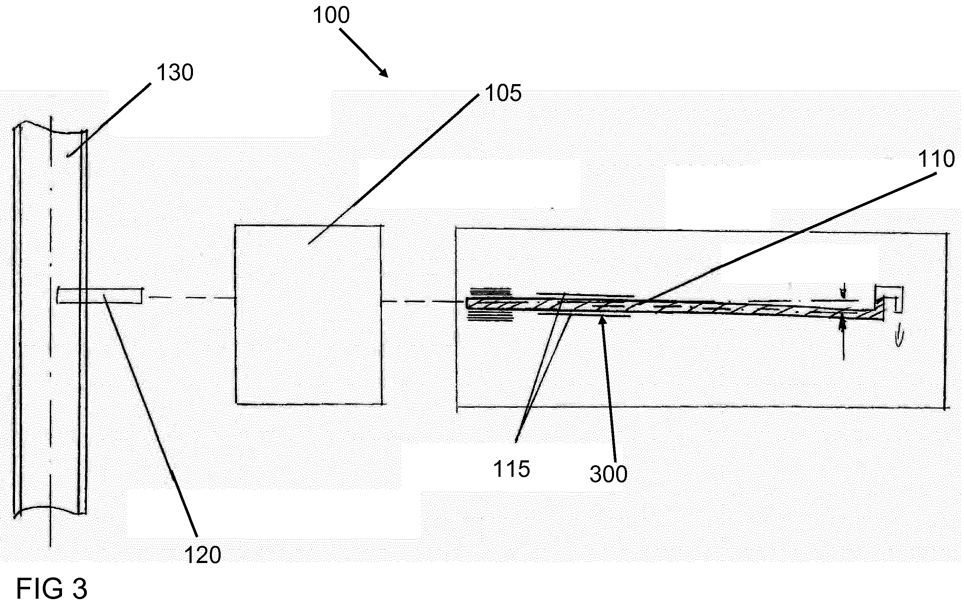

Figur 3 zeigt eine schematische Darstellung einer Analysevorrichtung 100 gemäß einem Ausführungsbeispiel. Dabei kann es sich um eine der anhand der Figuren 1 bis 2 beschriebenen Analysevorrichtungen 100 handeln, mit dem Unterschied, dass die Messeinrichtung 115 gemäß diesem Ausführungsbeispiel keine Wägezelle, sondern einen Dehnmessstreifen 300 umfasst.

Gemäß diesem Ausführungsbeispiel ist die einseitig eingespannte Aufnahmeeinrichtung 110 in Form des Messrohrs aus Glas ausgeformt und dazu ausgeformt, um beim Aufnehmen des Kondensat-Gas-Gemischs 135 eine Durchbiegung zu erfahren. Die Messvorrichtung 115 weist gemäß diesem Ausführungsbeispiel den Dehnmessstreifen 300 auf, der mit dem Messrohr gekoppelt ist und dazu ausgebildet ist, um abhängig von der Durchbiegung des Messrohrs die Gemischmasse anzuzeigen. Dies wiederum erlaubt die Berechnung des Gasanteils im Kondensat-Gas-Gemisch in Volumen-%. Die Justage der Messeinrichtung 115 kann mittels Prüfflüssigkeiten mit verschiedenen Dichten, z. B. durch vollentgastes Wasser und Prüföl, im Vorhinein vorgenommen worden sein.

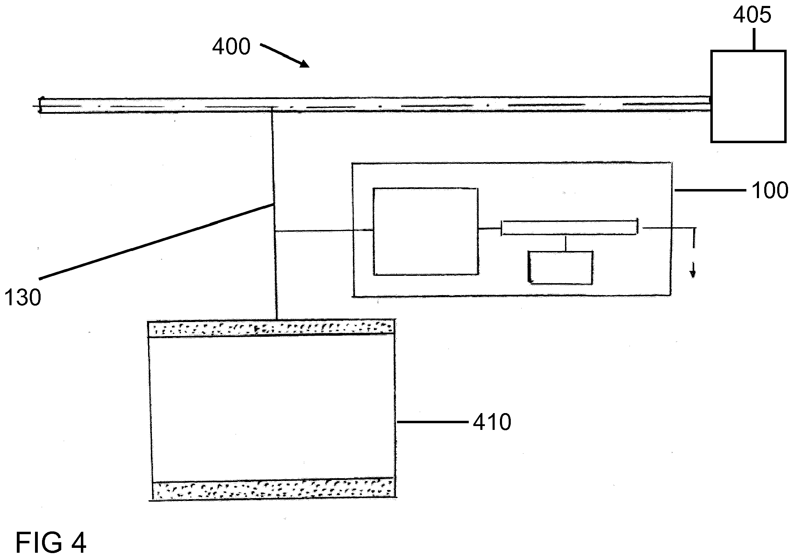

Figur 4 zeigt eine schematische Darstellung einer Sterilisiervorrichtung 400 gemäß einem Ausführungsbeispiel. Die Sterilisiervorrichtung 400 weist eine Dampferzeugungseinrichtung 405, einen Sterilisator 410 und eine der anhand der vorangegangenen Figuren beschriebenen Analysevorrichtungen 100 auf.

Die Dampferzeugungseinrichtung 405 ist dazu ausgebildet, um Dampf zu erzeugen, der über eine Dampfleitung 130 zu dem Sterilisator 410 geleitet wird. Die Dampfleitung 130 verbindet somit die Dampferzeugungseinrichtung 405 mit dem Sterilisator 410. Der Sterilisator 410 ist ausgebildet, um zu sterilisierende Gegenstände, beispielsweise Operationsbesteck, unter Verwendung des Dampfes zu sterilisieren. Die Analysevorrichtung 100 ist mit der Dampfleitung 130 gekoppelt und dazu ausgebildet, um einen Teil des Dampfes zur Analyse zu entnehmen, bevor der Dampf in den Sterilisator 140 eingeleitet wird.

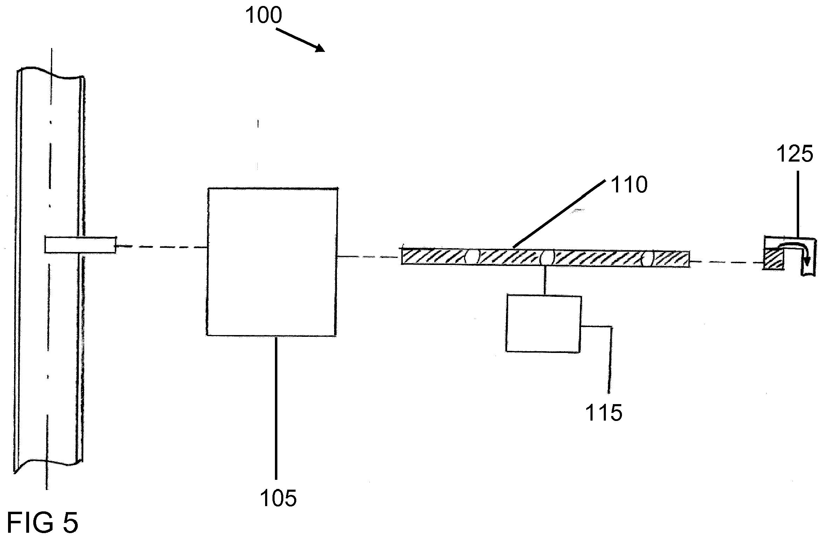

Figur 5 zeigt eine schematische Darstellung einer Analysevorrichtung 100 gemäß einem Ausführungsbeispiel. Dabei kann es sich um eine der anhand der vorangegangenen Figuren beschriebenen Analysevorrichtungen 100 mit einer Wägezelle handeln. Gemäß diesem Ausführungsbeispiel ist die als die Wägezelle ausgeformte Messeinrichtung 115 im Bereich einer Mitte der Aufnahmeeinrichtung 110 mit der Aufnahmeeinrichtung 110 gekoppelt. Im Unterschied zu dem anhand von Figur 2 beschriebenen Ausführungsbeispiel ist die Messeinrichtung 115 unterhalb der Aufnahmeeinrichtung 110 angeordnet. Gemäß einem Ausführungsbeispiel ist die Messeinrichtung 115 ausgebildet, um die Aufnahmeeinrichtung 110 von unten abzustützen.

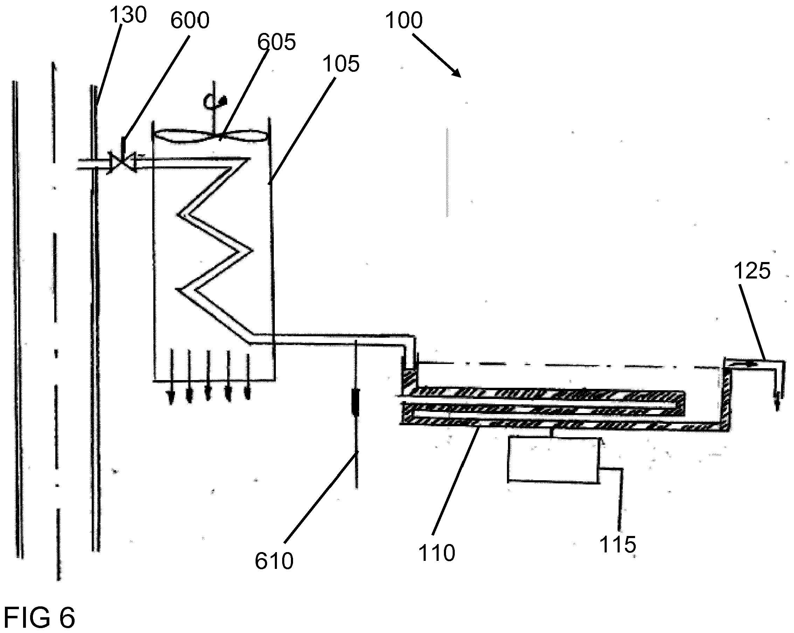

Figur 6 zeigt eine schematische Darstellung einer Analysevorrichtung 100 gemäß einem Ausführungsbeispiel. Dabei kann es sich um eine der anhand der vorangegangenen Figuren beschriebenen Analysevorrichtungen 100 mit der Wägezelle handeln.

Gemäß diesem Ausführungsbeispiel ist die Entnahmeeinrichtung als ein Entnahmeventil 600 ausgeformt und die Kühleinrichtung 105 weist zum Kühlen der Dampfmenge einen Ventilator 605 auf. Zwischen der Kühleinrichtung 105 und der Aufnahmeeinrichtung 110 weist die Analysevorrichtung 100 gemäß diesem Ausführungsbeispiel einen Temperatursensor 610 auf, der dazu ausgebildet ist, um eine Temperatur des Kondensat-Gas-Gemischs 135 zu sensieren. Die als das Messrohr ausgeformte Aufnahmeeinrichtung 110 ist gemäß diesem Ausführungsbeispiel aus Glas und spiralförmig ausgeformt. Dadurch ist eine kompakte Bauweise der Aufnahmeeinrichtung 110 realisierbar.

Im Folgenden werden Ausführungsbeispiele anhand der Fig. 6 nochmals näher beschrieben.

Gemäß diesem bevorzugten Ausführungsbeispiel wird die definierte Dampfmenge kontinuierlich aus der Dampfzuführung, in Form der Dampfleitung 130, zum Sterilisator mittels des Entnahmeventils 600 entnommen und über eine mittels dem Ventilator 605 gekühlte Kühlschlange kondensiert. Das Kondensat/Gas-Gemisch wird über eine Zuführungsleitung dem beispielsweise spiralförmigen Messrohr, hier beispielsweise aus Glas, mit definiertem Volumen zugeführt. Zur Berechnung der Temperaturkompensation wird die Temperatur des Kondensat/Gas-Gemischs in der Zuführungsleitung zwischen der Kühleinrichtung 105 und der Aufnahmeeinrichtung 110 mit einem mit der Steuerung des Sterilisators verbundenen Temperatursensor 610, vorzugsweise einem PT100, ermittelt. Das Gewicht des Kondensat/Gas-Gemischs wir mittels der Wägezelle 115 laufend erfasst. Die Wägezelle 115 ist mit der Steuerung des Sterilisators verbunden. Durch das bekannte Volumen und die bekannte Temperatur des Kondensats im Messrohr kann der Gasanteil einfach über die bekannte Volumen/Dichte-Beziehung berechnet werden.

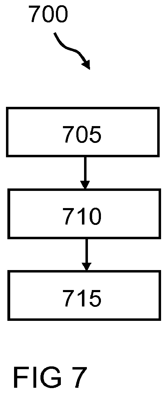

Figur 7 zeigt ein Ablaufdiagramm eines Verfahrens 700 zum Analysieren eines Dampfs zum Sterilisieren gemäß einem Ausführungsbeispiel. Dabei kann es sich um ein Verfahren 700 handeln, das von einer der anhand der vorangegangenen Figuren beschriebenen Analysevorrichtungen ausführbar ist.

In einem Schritt 705 des Kühlens wird eine Dampfmenge des Dampfs in einer Kühleinrichtung gekühlt zum Bewirken einer Kondensation zum Erhalten eines Kondensat-Gas-Gemischs. In einem Schritt 710 des Aufnehmens wird das Kondensat-Gas-Gemisch in eine Aufnahmeeinrichtung aufgenommen. In einem Schritt 715 des Messens wird eine Gemischmasse des Kondensat-Gas-Gemischs unter Verwendung einer mit der Aufnahmeeinrichtung gekoppelten Messeinrichtung gemessen.

## Patentansprüche

1. Analysevorrichtung (100) zum Analysieren eines Dampfs zum Sterilisieren für eine Sterilisiervorrichtung (400), wobei die Analysevorrichtung (100) zumindest die folgenden Merkmale umfasst:
eine Kühleinrichtung (105) zum Kühlen einer Dampfmenge des Dampfs zum Bewirken einer Kondensation zum Erhalten eines Kondensat-Gas-Gemischs (135);
eine Aufnahmeeinrichtung (110) zum Aufnehmen des Kondensat-Gas-Gemischs (135), wobei die Aufnahmeeinrichtung (110) mit der Kühleinrichtung (105) gekoppelt ist; und
eine Messeinrichtung (115) zum Messen einer Gemischmasse des Kondensat-Gas-Gemischs (135), wobei die Messeinrichtung (115) mit der Aufnahmeeinrichtung (110) gekoppelt ist.

2. Analysevorrichtung (100) gemäß Anspruch 1, mit einer Bestimmeinrichtung (140) zum Bestimmen eines Volumenanteils eines nichtkondensierbaren Gases des Kondensat-Gas-Gemischs (135) unter Verwendung der Gemischmasse und einer Sollmasse, wobei die Bestimmungseinrichtung (140) mit der Messeinrichtung (115) gekoppelt ist.

3. Analysevorrichtung (100) gemäß einem der vorangegangenen Ansprüche, mit einer Entnahmeeinrichtung (120) zum Entnehmen der Dampfmenge des Dampfs aus einer Dampfleitung (130) zum Führen des Dampfes in die Kühleinrichtung (105).

4. Analysevorrichtung (100) gemäß einem der vorangegangenen Ansprüche, bei der die Kühleinrichtung (105) zum Kühlen der Dampfmenge zumindest einen Ventilator (605) aufweist oder mit einem Ventilator (605) gekoppelt ist.

5. Analysevorrichtung (100) gemäß einem der vorangegangenen Ansprüche, bei der die Messeinrichtung (115) mit einer Steuerung der Sterilisiervorrichtung (400) gekoppelt oder koppelbar ist.

6. Analysevorrichtung (100) gemäß einem der vorangegangenen Ansprüche, bei der die Aufnahmeeinrichtung (110) zumindest ein Messrohr aufweist, das zumindest teilweise spiralförmig und/oder aus Glas ausgeformt ist.

7. Analysevorrichtung (100) gemäß einem der vorangegangenen Ansprüche, bei der die Messeinrichtung (115) zumindest eine Wägezelle aufweist, die dazu ausgebildet ist, um die Gemischmasse durch Wiegen zu messen.

8. Analysevorrichtung (100) gemäß einem der Ansprüche 1 bis 5, bei der die Aufnahmeeinrichtung (110) ein einseitig eingespanntes und durchbiegbares Messrohr aufweist.

9. Analysevorrichtung (100) gemäß Anspruch 8, bei der die Messeinrichtung (115) zumindest einen Dehnmessstreifen (300) aufweist, der dazu ausgebildet ist, um mittels einer Durchbiegung des Messrohrs die Gemischmasse anzuzeigen.

10. Analysevorrichtung (100) gemäß einem der vorangegangenen Ansprüche, mit einem Temperatursensor (610), der dazu ausgebildet ist, um eine Temperatur des Kondensat-Gas-Gemischs (135) zu sensieren, wobei der Temperatursensor (610) zwischen der Kühleinrichtung (105) und der Aufnahmeeinrichtung (110) angeordnet ist.

11. Sterilisiervorrichtung (400) zum Sterilisieren mittels Dampf mit einer Dampferzeugungseinrichtung (405) zum Erzeugen des Dampfs, einem Sterilisator (410) zum Verwenden des Dampfs, einer Dampfleitung (130) zum Verbinden der Dampferzeugungseinrichtung (405) mit dem Sterilisator (410) und einer Analysevorrichtung (100) gemäß einem der vorangegangenen Ansprüche, die mit der Dampfleitung (130) gekoppelt ist.

12. Verfahren (700) zum Analysieren eines Dampfs zum Sterilisieren für eine Sterilisiervorrichtung (400), wobei das Verfahren (700) zumindest die folgenden Schritte umfasst:
Kühlen (705) einer Dampfmenge des Dampfs in einer Kühleinrichtung (105) zum Bewirken einer Kondensation zum Erhalten eines Kondensat-Gas-Gemischs (135);
Aufnehmen (710) des Kondensat-Gas-Gemischs (135) in eine Aufnahmeeinrichtung (110); und
Messen (715) einer Gemischmasse des Kondensat-Gas-Gemischs (135) unter Verwendung einer mit der Aufnahmeeinrichtung (110) gekoppelten Messeinrichtung (115).
